# EUROPEAN PATENT APPLICATION

(11) **EP 4 620 473 A1**
(43) Date of publication of application: **24.09.2025**
(21) Application number: 23906714.3
(22) Date of filing: 05.12.2023
(51) Int. Cl.: A61K 31/7016, A23L 33/125, A61P 9/00, A61P 9/10

(54) **INHIBITOR OF INCREASE IN ARTERIAL STIFFNESS**

(30) Priority: 21.12.2022 JP 2022204343
(71) Applicant: Mitsui DM Sugar Co., Ltd., Tokyo 1080014 (JP)
(72) Inventor: KOBAYASHI Ryota, Tokyo 120-0045 (JP); SAKAZAKI Miki, Tokyo 161-0034 (JP); NAGAI Yukie, Tokyo 161-0034 (JP)
(74) Representative: Grünecker Patent- und Rechtsanwälte PartG mbB
(86) International application number: PCT/JP2023/043513
(87) International publication number: WO 2024/135348

(57) **Abstract**

The present invention relates to an inhibitor of an increase in arterial stiffness containing isomaltulose as an active ingredient, wherein the inhibitor of an increase in arterial stiffness is used such that the isomaltulose is continuously administered to a human once or more a day for 4 weeks or more.

## Description

### Technical Field

The present invention relates to an inhibitor of an increase in arterial stiffness.

### Background Art

Diseases related to blood vessels (cardiovascular diseases) such as heart disease and cerebrovascular disease are one of the major causes of death in Japan. Therefore, preventing cardiovascular diseases is an important challenge. Arterial stiffness, which refers to the physical stiffness of the arterial wall, has been reported to be a predictor of the onset of cardiovascular disease independent of blood pressure values. In order to prevent cardiovascular diseases, including arteriosclerosis (aging of blood vessels) and early vascular disorders, it is thus important to control not only the blood pressure value but also the arterial stiffness.

By inhibiting the increase in arterial stiffness, improvement and prevention of cardiovascular diseases are expected. Therefore, various studies have been made on components for inhibiting or improving arterial stiffness. For example, Patent Literature 1 discloses that cacao polyphenol is useful as an active ingredient of a composition for improving arterial stiffness.

### Citation List

### Patent Literature

Patent Literature 1: WO 2017/026471 A

### Summary of Invention

### Technical Problem

Postprandial hyperglycemia due to a meal containing a large amount of carbohydrates is said to be a risk factor for cardiovascular disease. One of the reasons for this is considered a decrease in vascular endothelial function through an increase in blood insulin levels associated with glucose load. Reduced vascular endothelial function due to the glucose load can result in increased arterial stiffness, a risk factor for cardiovascular disease. Therefore, for the prevention of cardiovascular diseases, it is important to inhibit the increase in arterial stiffness associated with postprandial hyperglycemia.

An object of the present invention is to provide an inhibitor of an increase in arterial stiffness, the inhibitor capable of inhibiting an increase in arterial stiffness even in a state where glucose is loaded.

### Solution to Problem

The present inventors have found that continuous ingestion of isomaltulose for a specific period of time can inhibit an increase in arterial stiffness even after ingesting a sugar that leads to a rise in blood glucose level and have completed the present invention.

One aspect of the present invention provides an inhibitor of an increase in arterial stiffness containing isomaltulose as an active ingredient, wherein the inhibitor of an increase in arterial stiffness is used such that the isomaltulose is continuously administered to a human once or more a day for 4 weeks or more.

Another aspect of the present invention provides an inhibitor of an increase in pulse wave velocity containing isomaltulose as an active ingredient, wherein the inhibitor of an increase in pulse wave velocity is used such that the isomaltulose is continuously administered to a human once or more a day for 4 weeks or more.

The inhibitor is preferably used such that the isomaltulose is administered in an amount of 20 g or more per administration.

A temporary effect of inhibiting the increase in arterial stiffness is less likely to be sustained, and the arterial stiffness usually decreases (deteriorates) to the same extent as the initial value after a certain period of time. On the other hand, since the inhibitor of an increase in arterial stiffness according to the present invention can inhibit an increase in arterial stiffness, when continuously administered, even after ingestion of a sugar (a sugar other than isomaltulose) that causes a rise in blood glucose level at a timing different from the ingestion of the inhibitor of an increase in arterial stiffness, it is considered that a functional and structural change of the arterial wall may occur.

### Advantageous Effects of Invention

According to the present invention, it is possible to provide an inhibitor of an increase in arterial stiffness, the inhibitor capable of inhibiting an increase in arterial stiffness even in a state where glucose is loaded.

### Brief Description of Drawings

FIG. 1 is a graph showing a transition of brachial-ankle pulse wave velocity (baPWV) in a test according to an example.
FIG. 2 is a result of statistical analysis on a transition of brachial-ankle pulse wave velocity (baPWV) in a test according to an example.

### Description of Embodiments

Hereinafter, embodiments of the present invention will be described. However, the present invention is not limited to the following embodiments.

An inhibitor of an increase in arterial stiffness according to the present embodiment contains isomaltulose as an active ingredient, and the inhibitor of an increase in arterial stiffness is used in such a manner that the isomaltulose is continuously administered to a human once or more a day for 4 weeks or more.

In the present specification, "arterial stiffness" refers to physical stiffness (physical flexibility) of an arterial wall. The arterial wall has a three-layer structure of the intima, media, and adventitia, and the arterial stiffness refers to the stiffness of the entire arterial wall, including all three layers, not to be evaluated from the stiffness of any of the layers. In addition, the arterial stiffness indicates the physical stiffness of the arterial wall, and is an index different from an atherogenic index calculated from cholesterol levels in blood.

The arterial stiffness can be evaluated by measuring, for example, pulse wave velocity (PWV) and cardio-ankle vascular index (CAVI), which are indicators of cardiovascular risk. PWV may be the brachial-ankle pulse wave velocity (baPWV), or heart-brachial pulse wave velocity (hbPWV). PWV and CAVI are higher as the degree of arterial stiffness is higher.

That is, the present invention can provide an inhibitor of an increase in pulse wave velocity containing isomaltulose as an active ingredient and can also provide an inhibitor of an increase in cardio-ankle vascular index containing isomaltulose as an active ingredient. The present invention can further provide an inhibitor of an increase in brachial-ankle pulse wave velocity and an inhibitor of an increase in heart-brachial pulse wave velocity, each of which contains isomaltulose as an active ingredient.

Isomaltulose is a compound formed by α-1,6 bonding of glucose and fructose, and is also referred to as 6-O-α-D-glucopyranosyl-D-fructose. Isomaltulose is also referred to as palatinose. Note that "PALATINOSE" is a registered trademark of Mitsui DM Sugar Co., Ltd.

Isomaltulose is found naturally in honey. It is also present in a transfer product generated when an α-glucosyltransferase (isomaltulose synthase) derived from bacteria or yeast acts on sucrose. Industrially, isomaltulose is produced by causing an α-glucosyltransferase derived from bacteria such as *Protaminobacter rubrum* and *Serratia plymuthica* to act on sucrose.

The isomaltulose used may be naturally derived or synthesized by an enzymatic action or the like.

Isomaltulose may be contained in the inhibitor of an increase in arterial stiffness as crystal particles or may be contained as granular particles. The granular particles may be, for example, (spherical) particles containing an aggregate of a plurality of crystal particles of isomaltulose and an amorphous sugar content, and the sugar content is contained in the aggregate of crystal particles. Such granular particles can be obtained, for example, by a method of precipitating crystal particles of isomaltulose from a sugar solution containing isomaltulose and an amorphous sugar content and spray-drying the sugar solution containing the crystal particles. Alternatively, granular particles containing isomaltulose can be obtained by a method of applying a shear force to the sugar solution while heating the sugar solution to precipitate crystal nuclei of isomaltulose and then cooling a mixture containing the crystal nuclei. The sugar solution can be obtained, for example, by causing an enzyme to act on sucrose. In this case, the sugar solution and the granular particles obtained contain trehalose, fructose, glucose, sucrose, isomaltose, and the like as amorphous sugar contents. The granular particles may be solid matters described in Japanese Unexamined Patent Publication No. 2012-179045 or the like.

In addition, commercially available isomaltulose may be used. Examples of such a commercially available product include crystalline palatinose (trade name: "Crystalline Palatinose PST-N", manufactured by Mitsui DM Sugar Co., Ltd.), powder palatinose (trade name: "Powder Palatinose PST-NP", manufactured by Mitsui DM Sugar Co., Ltd.), and palatinose syrup (trade names: "Palatinose Syrup-ISN" and "Palatinose Syrup-TN", manufactured by Mitsui DM Sugar Co., Ltd.).

The inhibitor of an increase in arterial stiffness according to the present embodiment only needs to contain isomaltulose as an active ingredient, and may be composed of isomaltulose alone, or may be a composition containing isomaltulose. When the inhibitor of an increase in arterial stiffness is a composition, isomaltulose contained therein may be contained as isomaltulose alone or contained as a commercially available isomaltulose preparation containing several saccharides. The isomaltulose preparation may contain an additional component in addition to isomaltulose.

The additional component may be a material that can be used for foods, quasi-drugs, or pharmaceuticals. The material that can be used for foods, quasi-drugs, or pharmaceuticals is not particularly limited, and examples thereof include an amino acid, a protein, a carbohydrate, a fat or oil, a sweetener, a mineral, a vitamin, a fragrance, an excipient, a binder, a lubricant, a disintegrant, an emulsifier, a surfactant, a base, a solubilizing agent, and a suspending agent.

Examples of the protein include milk casein, whey, soybean protein, wheat protein, and albumen. Examples of the carbohydrate include corn starch, cellulose, pregelatinized starch, wheat starch, rice starch, and potato starch. Examples of the oil include salad oil, corn oil, soybean oil, safflower oil, olive oil, and palm oil. Examples of the sweetener include saccharides such as glucose, sucrose, fructose, glucose-fructose syrup, and fructose-glucose syrup, sugar alcohols such as xylitol, erythritol, and maltitol, artificial sweeteners such as sucralose, aspartame, saccharin, and acesulfame K, and stevia sweeteners. Examples of the mineral include calcium, potassium, phosphorus, sodium, manganese, iron, zinc, magnesium, and salts thereof. Examples of the vitamin include vitamin E, vitamin C, vitamin A, vitamin D, vitamin B, biotin, and niacin. Examples of the excipient include dextrin, starch, lactose, and crystalline cellulose. Examples of the binder include polyvinyl alcohol, gelatin, hydroxypropyl methyl cellulose, hydroxypropyl cellulose, sodium carboxymethyl cellulose, and polyvinylpyrrolidone. Examples of the lubricant include magnesium stearate, calcium stearate, and talc. Examples of the disintegrant include crystalline cellulose, agar, gelatin, calcium carbonate, sodium hydrogen carbonate, and dextrin. Examples of the emulsifier or surfactant include sucrose fatty acid ester, citric acid, lactic acid, glycerin fatty acid ester, polyglycerin fatty acid ester, sorbitan fatty acid ester, propylene glycol fatty acid ester, and lecithin. Examples of the base include cetostearyl alcohol, lanolin, and polyethylene glycol. Examples of the solubilizing agent include polyethylene glycol, propylene glycol, sodium carbonate, and sodium citrate. Examples of the suspending agent include glyceryl monostearate, polyvinyl alcohol, polyvinyl pyrrolidone, methyl cellulose, hydroxymethyl cellulose, and sodium alginate. These may be used singly or in combination with two or more thereof.

Since isomaltulose has a sweetness of about 1/2 of sucrose, the inhibitor of an increase in arterial stiffness according to the present embodiment may mainly contain isomaltulose as a sweetener. Since the isomaltulose has a low hydrolysis rate of 1/5 of sucrose and is capable of inhibiting an increase in blood glucose level after ingestion, inclusion of isomaltulose mainly as a sweetener also produces an effect of facilitating blood glucose control.

When the inhibitor of an increase in arterial stiffness contains a component other than isomaltulose, the content of isomaltulose may be appropriately set according to the form, intended use, and the like of the inhibitor of an increase in arterial stiffness, but for example, may be 10 mass% or more, 20 mass% or more, or 30 mass% or more, and 70 mass% or less, 60 mass% or less, or 50 mass% or less with respect to the total amount of the inhibitor of an increase in arterial stiffness.

The inhibitor of an increase in arterial stiffness can be used as a food composition or a pharmaceutical composition. The food composition or pharmaceutical composition according to the present embodiment may be provided in the form of, for example, a health food, a food for specified health uses, a functional food, a nutraceutical food, a supplement, a quasi-drug, or a pharmaceutical. The food composition according to the present embodiment may be labeled, for example, to inhibit an increase in arterial stiffness and to improve the increased arterial stiffness. In addition, the form of the inhibitor may be any form such as a solid (powder, granules, etc.), a liquid (solution, suspension, etc.), or a paste and may be any dosage form such as a powder, a pill, a granule, a tablet, a capsule, a troche, a liquid, or a suspension.

The inhibitor of an increase in arterial stiffness according to the present embodiment is used such that isomaltulose is administered to a human once or more a day for 4 weeks or more.

The inhibitor of an increase in arterial stiffness is administered once or more a day from the viewpoint of producing an effect of inhibiting an increase in arterial stiffness even in a state where glucose is loaded. The inhibitor of an increase in arterial stiffness may be administered twice or more, or 3 times or more a day. The inhibitor of an increase in arterial stiffness may be administered up to 3 times a day.

The time of day when the inhibitor of an increase in arterial stiffness is administered is not particularly limited, but may be, for example, on an empty stomach before breakfast, simultaneously with breakfast, before lunch, or simultaneously with lunch.

The inhibitor of an increase in arterial stiffness is continuously administered for 4 weeks or more from the viewpoint of producing an effect of inhibiting an increase in arterial stiffness even in a state where glucose is loaded. The inhibitor of an increase in arterial stiffness may be administered for 8 weeks or more, or 12 weeks or more. The inhibitor of an increase in arterial stiffness may be administered for 52 weeks or less.

The inhibitor of an increase in arterial stiffness is preferably used such that isomaltulose is administered in an amount of preferably 20 g or more, more preferably 22 g or more, and still more preferably 25 g or more per administration. In addition, the inhibitor of an increase in arterial stiffness may be used such that isomaltulose is administered in an amount of 75 g or less, 72 g or less, or 70 g or less per administration. The inhibitor of an increase in arterial stiffness is preferably used such that isomaltulose is administered in an amount of preferably 20 g or more, more preferably 22 g or more, and still more preferably 25 g or more per day. In addition, the inhibitor of an increase in arterial stiffness may be used such that isomaltulose is administered in an amount of 75 g or less, 72 g or less, or 70 g or less per day. Within this range, a sufficient blood concentration can be achieved, and the action of inhibiting the increase in arterial stiffness can be exhibited more effectively.

The inhibitor of an increase in arterial stiffness may be administered orally or administered parenterally, such as intravenously. The inhibitor of an increase in arterial stiffness is preferably administered orally.

The subject to be administered with the inhibitor of an increase in arterial stiffness may be a human (healthy person) not suffering from diabetes. The human as an administration subject may be an elderly person of 60 years old or more, 65 years old or more, or 70 years old or more.

Specific aspects of the inhibitor of an increase in pulse wave velocity and inhibitor of an increase in cardio-ankle vascular index according to one embodiment may be the same as those of the above-described inhibitor of an increase in arterial stiffness. That is, the inhibitor of an increase in pulse wave velocity and inhibitor of an increase in cardio-ankle vascular index according to one embodiment may be obtained by replacing the "inhibitor of an increase in arterial stiffness" with the "inhibitor of an increase in pulse wave velocity" or the "inhibitor of an increase in cardio-ankle vascular index" in the above description regarding the inhibitor of an increase in arterial stiffness.

One embodiment of the present invention can also be regarded as a method for inhibiting an increase in arterial stiffness, pulse wave velocity, or cardio-ankle vascular index, the method including the step of using an effective amount of an inhibitor of an increase in arterial stiffness, an inhibitor of an increase in pulse wave velocity, or an inhibitor of an increase in cardio-ankle vascular index, each of which contains isomaltulose as an active ingredient, such that the isomaltulose is continuously administered to a human once or more a day for 4 weeks or more. Aspects, administration methods, dosages, and the like of the inhibitor of an increase in arterial stiffness, the inhibitor of an increase in pulse wave velocity, and the inhibitor of an increase in cardio-ankle vascular index may be the same as those described above. In addition, one embodiment of the present invention can be regarded as isomaltulose for use in a method for inhibiting an increase in arterial stiffness, pulse wave velocity, or cardio-ankle vascular index, wherein the isomaltulose is continuously administered to a human once or more a day for 4 weeks or more.

One embodiment of the present invention can also be regarded as the use of isomaltulose in the production of an inhibitor of an increase in arterial stiffness, an inhibitor of an increase in pulse wave velocity, or an inhibitor of an increase in cardio-ankle vascular index. Aspects of the inhibitor of an increase in arterial stiffness, the inhibitor of an increase in pulse wave velocity, and the inhibitor of an increase in cardio-ankle vascular index may be the same as those described above. In addition, one embodiment of the present invention can be regarded as the use of isomaltulose in inhibiting an increase in arterial stiffness, pulse wave velocity, or cardio-ankle vascular index, wherein the isomaltulose is continuously administered to a human once or more a day for 4 weeks or more.

### Examples

Hereinafter, the present invention will be described in more detail based on Examples. However, the present invention is not limited to the following Examples.

According to the following method, the influence of long-term intake of isomaltulose on increased arterial stiffness associated with postprandial hyperglycemia was examined.

### <Subject>

Subjects were 54 healthy middle to elderly persons (30 males and 24 females). All subjects were assigned to one of the groups, i.e., an isomaltulose ingestion group (Group I) and a sucrose ingestion group (Group S), in a single blind test before intervention, and the intervention was performed for 12 weeks each.

### <Test Design>

All subjects ingested jellies having compositions described in Table 1 once a day for 12 weeks continuously. Group I received a jelly containing isomaltulose (Example 1), and group S received a jelly containing sucrose (Comparative Example 1). The jelly was taken every morning before breakfast, and all subjects were instructed not to change their dietary habits during the intervention period from those before the intervention. A 75 g oral glucose tolerance test (75-g OGTT, described in detail below) was performed before the 12-week intervention, and at 4 weeks, 8 weeks, and 12 weeks after the start of the intervention, after the lapse of 1 day or more from the ingestion of the jelly and the arterial stiffness was evaluated by the method described below before the 75-g OGTT and at 60 minutes and 120 minutes after the 75-g OGTT.

**[Table 1]**

| Jelly composition (g) | Comparative Example 1 | Example 1 |
|---|---|---|
| Gelling agent | 0.7 | 0.7 |
| Sucrose | 25 | - |
| Isomaltulose | - | 25 |
| Acidulant (50% citric acid) | 0.2 | 0.2 |
| Fragrance | 0.2 | 0.2 |
| Defoamer | 0.03 | 0.03 |
| Water | 73.87 | 73.87 |
| Total | 100 | 100 |

### <75 g Oral Glucose Tolerance Test (75-g OGTT)>

The 75-g OGTT was performed under the instruction of a doctor using TRELAN- G 75 g (Yoshindo Inc.), which is generally used in medical institutions and research, and the test solution was orally ingested in a general volume (225 mL per dose) within 5 minutes according to the guidelines of the Japan Diabetes Society (Araki E, Goto A, Kondo T, Noda M, Noto H, Origasa H, et al. Japanese Clinical Practice Guideline for Diabetes 2019. J Diabetes Investig 2020; 11: 1020-1076.).

### <Evaluation of Arterial Stiffness>

The arterial stiffness was evaluated by calculating the pulse wave velocity (PWV) from the pulse transit time between two points in the artery and the distance measured with a measuring tape (PWV = length of artery/pulse transit time). The brachial-ankle pulse wave velocity (baPWV) was evaluated as an index of the systemic arterial stiffness by attaching oscillometric sensors to the left and right upper arms and ankles using a blood pressure pulse wave inspection apparatus (BP-203RPEII, from FUKUDA COLIN Co., Ltd.). The intra-examiner and inter-examiner coefficients of variation for measured values of PWV were 3% and 4%.

### <Statistical Processing>

A change in baPWV before and after the start of the intervention of each group was shown as the average value (95% confidence interval). For the comparison of the change in baPWV between the 2 groups before and after the 75-g OGTT, a two-way repeated measures ANOVA was used. The Bonferroni method was performed for comparison of changes in each intervention in post-hoc test. The total area under the curve at 90 minutes (baPWV AUC) was calculated using the trapezoidal rule. For statistical analysis, IBM SPSS Statistics Ver. 25 (manufactured by IBM Corporation) was used. The statistical significance level in this test was set at 5%.

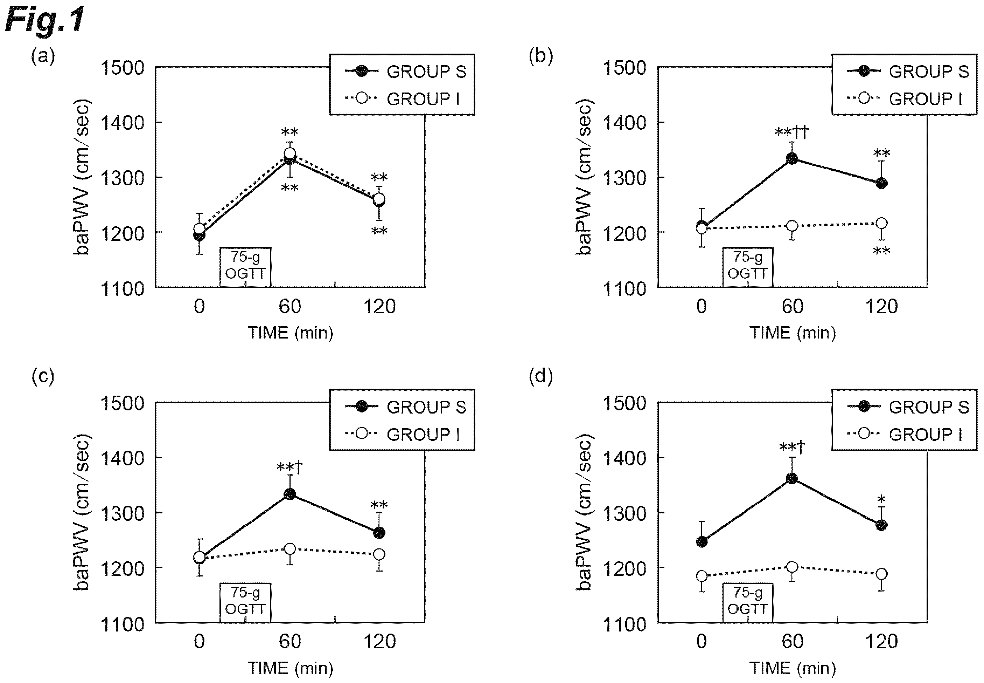

FIG. 1 shows the transition of baPWV of each group before and after the start of the intervention. FIG. 1(a) shows the transition of baPWV before the intervention and shows the transition of baPWV before the 75-g OGTT, at 60 minutes after the 75-g OGTT, and at 120 minutes after the 75-g OGTT. FIGS. 1(b), (c), and (d) show baPWV at 4, 8, and 12 weeks after the start of the intervention, respectively, and show the transition of baPWV before the 75-g OGTT, at 60 minutes after the 75-g OGTT, and at 120 minutes after the 75-g OGTT.

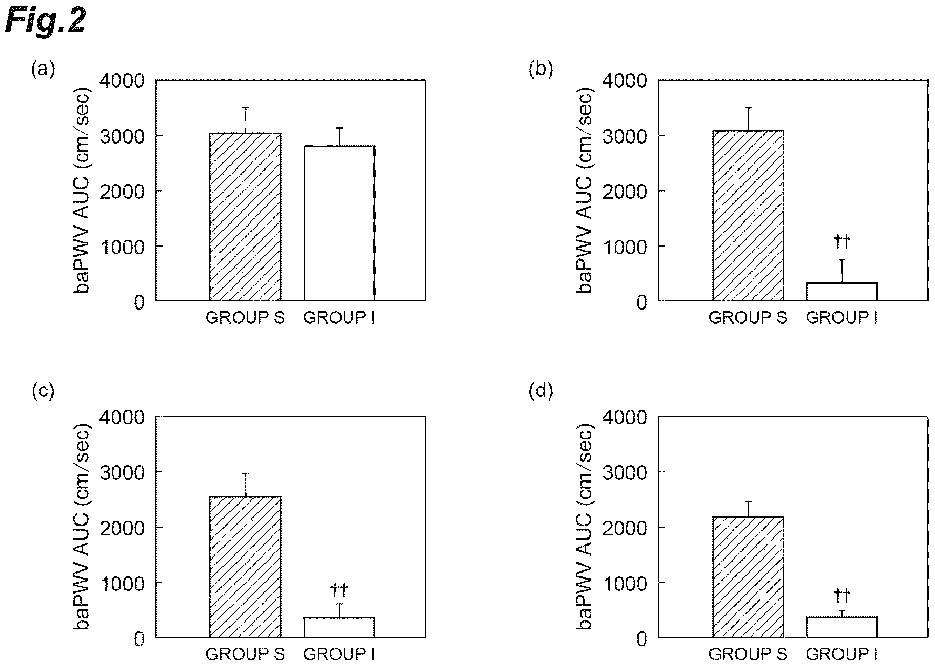

FIG. 2 shows baPWV AUC for each group before and after the start of the intervention. FIGS. 1(a), (b), (c), and (d) show the baPWV AUC for each group before the intervention, and at 4, 8, and 12 weeks after the start of the intervention, respectively.

The baPWV at 4, 8, and 12 weeks after the start of the intervention significantly increased in group S at 60 minutes (p < 0.05) and 120 minutes (p < 0.05) after the 75-g OGTT compared to before the 75-g OGTT, but no significant change was found in Group I. On the other hand, the baPWV at 60 minutes after the 75-g OGTT showed a significantly lower value at 4, 8, and 12 weeks after the start of the intervention in Group I than in Group S (p < 0.05). No change in baPWV before the 75-g OGTT was found after intervention compared to before intervention in both groups. No group difference was observed in baPWV AUC before the intervention. The baPWV AUC at 4, 8, and 12 weeks after the start of the intervention showed a significantly lower value in Group I than in Group S (p < 0.01). As described above, the baPWV at 60 minutes and 120 minutes after the 75-g OGTT was lower in Group I than in Group S at 4, 8, and 12 weeks after the start of the intervention, indicating that the increase in arterial stiffness after glucose load was inhibited.

## Claims

1. An inhibitor of an increase in arterial stiffness, comprising:
isomaltulose as an active ingredient, wherein
the inhibitor of an increase in arterial stiffness is used such that the isomaltulose is continuously administered to a human once or more a day for 4 weeks or more.

2. An inhibitor of an increase in pulse wave velocity, comprising:
isomaltulose as an active ingredient, wherein
the inhibitor of an increase in pulse wave velocity is used such that the isomaltulose is continuously administered to a human once or more a day for 4 weeks or more.

3. The inhibitor according to claim 1 or 2, wherein the inhibitor is used such that the isomaltulose is administered in an amount of 20 g or more per administration.
